# EUROPEAN PATENT APPLICATION

(11) **EP 3 895 601 A1**
(43) Date of publication of application: **20.10.2021**
(21) Application number: 20169254.8
(22) Date of filing: 14.04.2020
(51) Int. Cl.: A61B 5/00, A61B 5/1455, A61B 5/053, A61B 5/20

(54) **SYSTEM AND METHOD FOR MONITORING HYDRATION STATUS OF A HUMAN BODY**

(71) Applicant: Stichting IMEC Nederland, 5656 AE Eindhoven (NL)
(72) Inventor: Groenendaal, Willemijn, 3001 Leuven (BE); Lee, Seulki, 3001 Leuven (BE)
(74) Representative: Körfer, Thomas

(57) **Abstract**

A system (10) for monitoring hydration status of a human body (1) is provided. The system (10) comprises a plurality of sensors (11₁, 11₂, 11₃, 11₄) adapted to perform bio-impedance measurements at predefined locations on the body (1), thereby generating respective measurement data. The system (10) further comprises a processing means (13) adapted to receive and to process the measurement data in order to estimate a body composition parameter for the respective locations on the body (1) . In this context, at least one sensor (11₁) is adapted to perform bio-impedance measurement at the throat region or esophagus region (2) in order to detect a swallowing or fluid intake event.

## Description

The invention relates to monitoring the hydration status of a human body, especially for modelling a fluid map for the whole body manifesting the level of fluid intake, distribution and output for a given monitoring period.

Generally, bio-impedance (BioZ) is a measure for to what extend tissue impedes an electric current flow. Each bioZ measurement generates two parameters: the resistance and the reactance. Resistance is a measure of the obstruction to an electrical current by different tissues, while reactance is related to the electrical current storage. In human tissues, resistance is mostly determined by intra- or extracellular body water, while reactance is influenced by the capacitance of the cell membranes. The frequency of the alternating current (expressed as kHz) determines which compartment of the human body is assessed. When a low frequency alternating current is applied to the human body, it cannot penetrate cell membranes so bioZ will mainly contain information of the bioZ_{R} of the extracellular fluid compartment. In contrast, a high frequency alternating current can penetrate the cell membranes, so the intracellular fluid volumes and the cell membranes will contribute to the bioZ signal as well as the extracellular fluid compartment.

For example, the document US 8,406,865 B2 discloses a bio-impedance system comprising a sensor assembly for bio-impedance measurements to provide information related to the lean body water of the patient's tissue. The information related to the patient's lean body water may be determined by spectroscopic methods for determining water fraction. However, in order to perform continuous monitoring, all sensors are required to be active throughout the monitoring duration, which degrades the effective operation lifespan of the sensors (e.g., battery life) especially for remote sensors. Moreover, the measurements are performed only to estimate the current status of the body water, whereas the estimation of fluid intake is an essential parameter to be measured in order to model a fluid map for the whole body. In addition, an estimation of fluid output facilitates real-time assessment or at least fast assessment (i.e., not necessarily true real-time assessment) for the fluid map, which allows feedback to the user.

Accordingly, the object of the invention is to provide a system, a method and a computer program for continuous monitoring of hydration status of a human body, which can address the aforementioned limitations.

The object is solved by the features of the first independent claim for the system and by the features of the second independent claim for the method. The dependent claims contain further developments and the computer program.

According to a first aspect of the invention, a system for monitoring hydration status of a human body is provided. The system comprises a plurality of sensors adapted to perform bio-impedance measurements at predefined locations on the body, thereby generating respective measurement data. The system further comprises a processing means adapted to receive and to process the measurement data in order to estimate a body composition parameter for the respective locations on the body. In this context, at least one sensor is performing bio-impedance measurement at the throat region or esophagus region in order to detect a swallowing or fluid intake event. Moreover, the processing means is further adapted to control the rest of the sensors based on the detected swallowing or fluid intake event.

Herein, the estimation of body composition parameter includes but is not limited to the estimation of fluid volume, fat mass, lean mass or a combination thereof. Therefore, a multi-sensor based monitoring approach is provided for monitoring the hydration status of the whole body using a network of sensors. The locations of the sensors resp. of the body compartments are selected on a case-by-case basis, whether the patient is a healthy individual or is being treated for chronic health conditions. For instance, important body compartments for a congestive heart failure (CHF) patient to be monitored are pulmonary area and peripheral area (chest and leg/ankle) .

Moreover, a swallowing or fluid intake detection mechanism is incorporated in order to control the sensors either centrally (e.g., master-slave configuration) or remotely (e.g., by a user through the processing means). It is advantageous that one sensor is dedicated for measuring bio-impedance at the throat region or at esophagus region. The absolute value of the measured bio-impedance completely describes the swallowing process, i.e. the closure of the larynx. There is a typical reproducible drop in bio-impedance during a swallow and a simple valley detection can sense a respective swallowing action. In addition, a significant change in impedance can be observed at the esophagus tube during fluid intake, since the walls of the esophagus squeeze or contract together to move the fluid down the esophagus to the stomach.

According to a first preferred implementation form of said first aspect of the invention, at least one sensor is adapted to perform bio-impedance measurement at a predefined location capable of capturing the impedance changes of the stomach, thereby generating measurement data for estimating a fluid volume in the stomach. For instance, the sensor may perform bio-impedance measurement at the upper abdomen region or at the back to generate measurement data for estimating a fluid volume or volume change in the stomach.

Furthermore, at least one sensor is performing bio-impedance measurement at a predefined location capable of capturing the impedance changes of the bladder, thereby generating measurement data for estimating a fluid volume in the bladder. For example, the sensor may perform bio-impedance measurement at the lower abdomen region or at the back to generate measurement data for estimating a fluid volume in the bladder. Thus, the system accordingly estimates the volume of water intake (e.g., stomach) as well as the volume of water output (e.g., bladder) for a given monitoring period in order to model the fluid map for the whole body.

Preferably, the plurality of sensors are similar in terms of their data acquisition, data transmission and internal processing. However, it is further conceivable that the plurality of sensors can be different in terms of their firmware configuration. It is also conceivable that the plurality of sensors may differ in terms of their hardware configuration or form factor.

According to a further preferred implementation form of said first aspect of the invention, the plurality of sensors are interchangeable with respect to their respective locations on the body. Advantageously, a high degree of reproducibility can be achieved to the extent where consistent results can be obtained when the measurements are repeated for different body locations. In addition to this or as an alternative, the plurality of sensors are integrated in clothing in order to link the plurality of sensors to their respective locations on the body.

According to a further preferred implementation form of said first aspect of the invention, the system is operable in an idle mode where at least one sensor is continuously active and the rest of the sensors are in a sleep mode. In this respect, the continuously active sensor preferably performs bio-impedance measurement at the throat region or esophagus region and is further adapted to transmit a trigger signal towards the processing means upon detecting a swallowing or fluid intake event. Moreover, the processing means is further adapted to generate control signals based on the trigger signal in order to perform wakeup control of the sensors that are in sleep mode.

Therefore, the system requires only one sensor to be continuously active and the rest of the sensors remain inactive or in sleep mode. This advantageously facilitates a prolonged operational lifespan for the sensors resp. for the monitoring system. The inactive sensors are switched back on upon detecting a swallowing or fluid intake event. It is possible to trigger the inactive sensors by the continuous active sensor instead of mediating the trigger signal through the processing means in order to perform wake up control, thereby facilitating a master-slave configuration.

In addition to the foregoing arrangement, the system preferably performs a scheduled wake-up operation for the sensors that are in sleep mode, for instance, wake-up recurrences predefined by a caregiver inputted into the system. This facilitates occasional measurement at the predefined locations, independent of the wake-up trigger signal (either positive or false positive). Hence, the system advantageously configures the sensors (especially the sensors measuring TBW) to wake-up occasionally and to perform measurements, so that the caregiver is still able to monitor the dehydration process of a patient, even if the patient does not drink for a long period of time.

Furthermore, the processing means additionally performs corrections for any false positive trigger detection, e.g., a detected swallowing event without actually drinking. In this context, upon detecting a swallowing event and successively waking up the inactive sensors, the processing means performs a check whether the sensor at the stomach detects a change within a specified period. If no substantial change is recorded at the stomach, the measurement procedure is halted and the system resumes back to its idle mode where only the sensor at the throat region or at the esophagus region is active and the rest of the sensors are inactive.

According to a further preferred implementation form of said first aspect of the invention, the processing means is further adapted to configure the plurality of sensors based on a predefined time settings in order to define a measurement duration and a measurement rate of recurrence for the respective sensors. Hence, in addition to the fluid input and fluid distribution estimations, the system further takes into account delays between input and distribution, thereby improving the monitoring reliability significantly.

Moreover, the processing means is further adapted to configure the plurality of sensors in a manner that the plurality of sensors are not active at the same time, especially when current paths of the plurality of sensors can get mixed. This can be achieved by defining specific measurement duration and specific measurement rate of recurrence for the respective sensors or by operating the plurality of sensors at different settings (e.g., firmware, form factor, and the like).

According to a further preferred implementation form of said first aspect of the invention, the plurality of sensors are further adapted to transmit the respective measurement data to the processing means upon detecting a variation with respect to a reference value or to a value from an immediate preceding measurement. Therefore, in addition to trigger based data collection, the system further facilitates continuous data collection from the sensors, which requires very little to no bi-directional communication between the sensors and the processing means.

From this perspective, all sensors are configured for a specific duration of monitoring period at each respective body locations, preferably by the processing means before commencing the monitoring phase. As such, the sensors are continuously monitoring the respective body locations and the measurement data are only sent to the processing means once a change is observed. For this purpose, a simple on-sensor processing can be implemented, e.g., by means of an if statement, with respect to a predefined threshold.

According to a further preferred implementation form of said first aspect of the invention, the system further comprises at least one additional sensor adapted to perform galvanic skin response measurement and/or sweat composition measurement at a predefined location on the body, thereby generating measurement data for fluid loss estimation. For instance, the additional sensor can be a sweat patch that collects sweat at the surface of the skin and can measure different compositions, e.g., sodium, potassium, and glucose. Furthermore, a fluid rate can also be estimated that provides additional insights on the rate of fluid loss.

Additionally or alternately, the system further comprises at least one additional sensor adapted to perform photoplethysmogram measurement at a predefined location on the body, thereby generating measurement data in order to increase the accuracy for the fluid estimation. Advantageously, additional physiological parameters, e.g., galvanic skin response (GSR) for sweat monitoring, photoplethysmogram (PPG) for blood volume monitoring, and the like, are taken into consideration in order to improve the fluid distribution and output estimations.

According to a further preferred implementation form of said first aspect of the invention, the processing means is implemented on a cloud environment. In addition, the system comprises a user interface and whereby the processing means is further adapted to configure the plurality of sensors based on control commands received from a user via the user interface. Hence, the high degree of mobility and reliability offered by cloud-based processing are advantageously incorporated. Furthermore, the processing means is able to send feedback to the user based on the processed data/results from respective measurements. Such a user can be an individual or a caregiver, where the feedback can be a trend or the actual values/thresholds. Further, the feedback can be actions or actionable insights, for instance, the patient is required to drink, the patient is required to contact the caregiver, detection of fluid overload, the patient is required to take medications, and the like.

According to a second aspect of the invention, a method for monitoring hydration status of a human body is provided. The method comprises the step of performing bio-impedance measurements at predefined locations on the body by a plurality of sensors, thereby generating respective measurement data. The method further comprises the step of receiving and processing the measurement data by a processing means in order to estimate a body composition parameter for the respective locations on the body. In this regard, at least one sensor is adapted to perform bio-impedance measurement at the throat region or esophagus region in order to detect a swallowing or fluid intake event. Therefore, a multi-sensor based monitoring approach is provided for monitoring the hydration status of the whole body using a network of sensors.

Exemplary embodiments of the invention are now further explained with respect to the drawings by way of example only, and not for limitation. In the drawings:
- Fig. 1: shows the prospective body locations for compartment based monitoring approach by way of an example;
- Fig. 2: shows an exemplary embodiment of the system according to the first aspect of the invention;
- Fig. 3: shows an exemplary process flow chart for controlling the sensors;
- Fig. 4: shows an exemplary model for measurement data processing and feedback;
- Fig. 5A: shows an exemplary correlation analysis of Thorax measurement for a heart failure patient;
- Fig. 5B: shows an exemplary correlation analysis of Foot measurement for a heart failure patient; and
- Fig. 6: shows an exemplary embodiment of the method according to the second aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

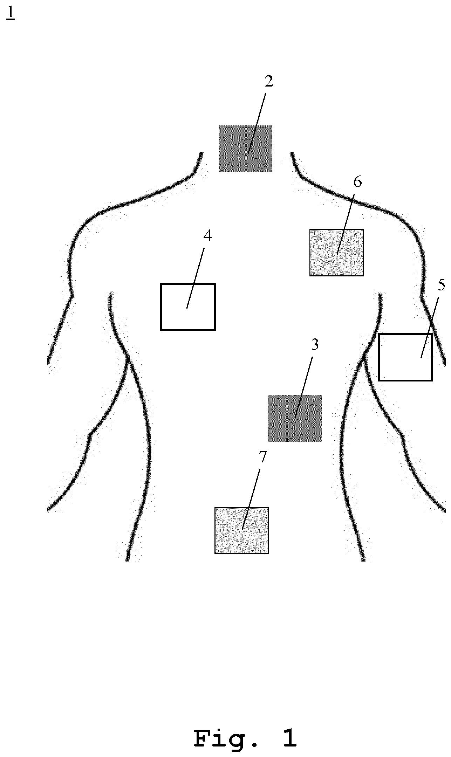

In Fig. 1, the prospective body locations for compartment based monitoring approach are illustrated by way of an example. Considering the frequency response of bio-impedance and the dielectric/conductive properties of the different tissues, it is possible to estimate the diverse compartments of the human body, i.e., intracellular and extracellular water, lean mass, fat mass, etc. Measurement of total body bio-impedance is the most commonly used method for estimating whole body compartments. Many of the whole body bio-impedance instruments apply three approaches for impedance measurement: hand-to-foot method, foot-to-foot method and hand-to-hand method. The hand-to-foot one is the most commonly used method.

The aforementioned methods perform either a single measurement (e.g., whole body measurement) or multiple measurements (e.g., segmented approach) in order to estimate the total body water or composition. A continuous monitoring of current fluid status of a body cannot be achieved by a single whole body measurement or by the traditional segmented approach. With the idea of having sensors performing bio-impedance measurement, which can be placed anywhere on the body, the proposed invention introduces a novel compartment based monitoring in order to monitor the fluid status of the body continuously and further allowing feedback to the user.

Therefore, instead of measuring whole body bio-impedance (e.g., the hand-to-foot method), the invention proposes to perform multiple localized bio-impedance measurements by using multiple sensors. Each sensor will give localized fluid information, and the combined sensor network will provide overall fluid information and actionable information.

For example, the perspective locations for bio-impedance measurements on the body 1 can be defined based on patient's health conditions. In general, fluid intake can be estimated from measurements performed at throat region or at esophagus region 2 and at upper abdomen region or stomach 3. The volume of fluid output can be estimated from measurements performed at lower abdomen region or bladder 7. Additionally, with the help of galvanic skin response (GSR) and/or sweat composition measured at any location on the skin, e.g., 6, fluid loss through sweat can also be estimated.

These additional measurements provide a complete picture of the overall estimation for fluid output. In order to monitor the current fluid status, bio-impedance measurements can be performed at torso 4 as well as at peripheries 5. Hence, by means of the proposed compartment based measurement, multiple measurements on multiple locations of the body 1 can be performed, preferably in a sequence, thereby facilitating a continuous monitoring of the fluid status of the whole body 1.

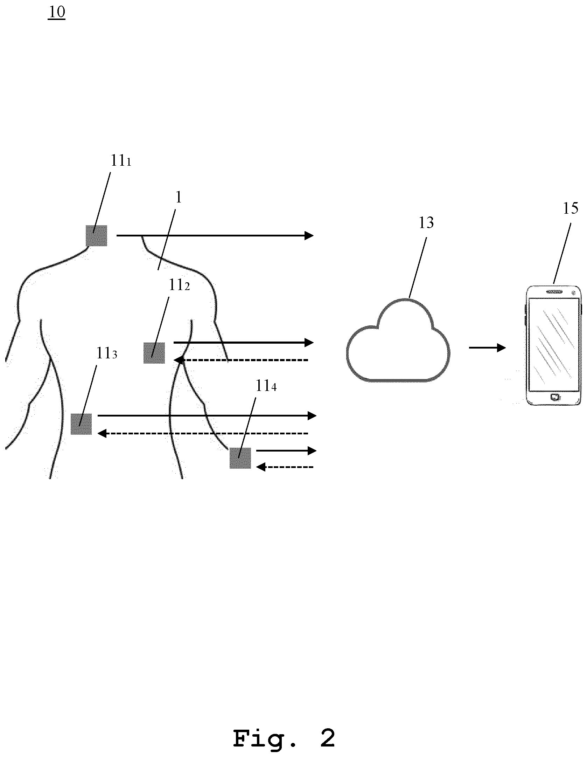

In Fig. 2, an exemplary embodiment of the system 10 according to the first aspect of the invention is illustrated. The system 10 comprises a plurality of sensors 11₁, 11₂, 11₃, 11₄ that perform bio-impedance measurement at specific locations on the body 1, thereby forming a sensor network. The system 10 further comprises a processing means 13 that is able to communicate with the sensors 11₁,11₂,11₃,11₄, preferably in a bi-directional manner. The sensors 11₁, 11₂, 11₃, 11₄ are preferably remote sensors, where the processing means 13 communicates with the sensors 11₁, 11₂, 11₃, 11₄ wirelessly.

It is advantageous that the processing means 13 is realized in a cloud environment in order to incorporate the high degree of mobility and reliability of cloud based processing to access and to further process the measurement data. A user interface 15 is also incorporated into the system 10, through which a user can manipulate or expedite the measurement configuration as well as processing. The user interface 15 can be a smartphone, where the user can configure the system 10, e.g., via an application. Additionally or alternately, the user interface 15 can be a web-based graphical user interface (web-GUI), executable in a personal computer where the computer is connected to the cloud via internet.

As already mentioned above, the prospective measurement locations of the body are defined on the basis of a patient's health condition. For instance, a patient with congestive heart failure, measurements at pulmonary area and peripheral area provides a complete estimation of accumulated fluid in both lung and peripherals. Upon determining the locations, a user may link the sensors 11₁, 11₂, 11₃, 11₄ and the respective measurement locations through the user interface 15, e.g., via the application. Another possible implementation of the sensor network includes the integration of the sensors 11₁,11₂,11₃,11₄ in clothing, either with adapters/holders on the clothing or fully integrated in clothing, thereby linking the sensors 11₁,11₂,11₃,11₄ to the positions automatically.

In one implementation, the processing means 13 is able to collect measurement data continuously from the sensors 11₁,11₂,11₃,11₄. Depending on the sensor location, measurement instances and intervals could be every 5-10 minutes for approximately 5-60 seconds. Particularly for this implementation, the processing means 13 is not required to communicate bi-directionally with the sensors 11₁, 11₂, 11₃, 11₄. In this regard, the sensors 11₁,11₂,11₃,11₄ are incorporated with simple on-sensor processing to detect a change in the measured data, either respect to a defined threshold or to the data from immediate preceding measurement. A simple if-statement would suffice to implement the detection algorithm. As a consequence, the sensors 11₁,11₂,11₃,11₄ will only send the measurement to the processing means 13 only when a change is observed.

In a preferred implementation, the processing means 13 initiates the sensors 11₁,11₂,11₃,11₄ to perform bio-impedance measurements, e.g., for 60 seconds, at the respective locations in order to collect initial measurement data as base or reference values. After the initial data collection, all but one sensor 11₁ of the plurality of sensors 11₁, 11₂, 11₃, 11₄ remain in a sleep mode. This configuration is hereafter referred to as the idle mode. During this mode of operation, one sensor or the throat sensor 11₁ remains active and continuously monitors at the throat region or at esophagus region 2. The throat sensor 11₁ preferably performs bio-impedance measurement at throat or at esophagus region in order to detect a swallowing or fluid intake event.

Upon detecting a swallowing or fluid intake event, e.g., by means of valley detection or by detecting contraction at the esophagus tube mentioned before, the throat sensor 11₁ transmits a trigger signal towards the processing means 13. In response, the processing means 13 generates control signals for wakeup control of the rest of the sensors 11₂,11₃,11₄, thereby initiating bio-impedance measurements at other body locations. The sensors 11₁, 11₂, 11₃, 11₄ are further configured with respect to the measurement duration and the measurement rate of recurrence, preferably by a user through the processing means, so as to take the delays between fluid input and distribution into account.

For instance, upon initiating the sensors 11₂,11₃,11₄ after detecting a swallowing or fluid intake event, data collection starts immediately at the stomach. In the beginning, the measurement rate of recurrence at the stomach is approximately every 10 seconds, later every 5 minutes and the measurement duration is typically 2 hours. Other sensors are also triggered immediately after the detection of a swallowing or fluid intake event, where data collection is performed in every 5-10 minutes. In this case, the processing means 13 communicates with the sensors 11₁,11₂,11₃,11₄ in a bi-directional manner and based on the trigger signal, the processing means 13 sends control signal to dedicated sensors, which will then start/stop data collection with these sensors 11₁, 11₂, 11₃, 11₄.

Alternately, it is further possible to configure the sensor network in a master-slave fashion, where the throat sensor 11₁ operates as the master sensor and the rest are slave sensors. Upon detecting a swallowing or fluid intake event, instead of sending the trigger signal to the processing means 13, the throat sensor 11₁ uses the trigger signal for wakeup control of the rest of the sensors 11₂,11₃,11₄, thereby initiating measurements at different body locations. In this case, the processing means 13 is not exclusively required to communicate with the sensors 11₁,11₂,11₃,11₄ in a bi-directional manner, especially if a user externally provides the base or reference values.

The processing means 13 is further able to send feedback to the user based on the processed data/results from respective measurements. Such a user can be an individual or a caregiver, where the feedback can be a trend or the actual values/thresholds. Such feedback can be categorized into short-term feedback and long-term feedback. Examples of short-term feedback may include health status of the heart failure patient for acute trend monitoring. The basis of this is to monitor fluid removal from the lungs or peripherals based on a change in treatment or follow up of the buildup of fluid after treatment changes.

Another example of short-term feedback may regard to monitoring of the total body water to provide feedback to the user if the subject is over-hydrated or dehydrated. Especially for kidney patients, this could relate to dialysis treatment or the amount of water they can still drink. Athletes or elderly persons can also use this to monitor dehydration or over-hydration. Examples of long-term feedback may include long-term trend monitoring in disease progression, either based on the time constants of transport between compartments or based on the status of one compartment.

Furthermore, in addition to the bio-impedance measurement sensors 11₁, 11₂, 11₃, 11₄, the sensor network preferably comprises additional sensors for estimating different physiological parameters. For example, a galvanic skin response (GSR) measurement sensor can provide measurement data from skin response in order to estimate fluid loss through sweat. A sweat patch can collect sweat at the surface of the skin and can measure different compositions, thereby providing measurement data from sweat analysis in order to estimate fluid loss through sweat. A photoplethysmogram (PPG) measurement sensor can provide insights on blood volume changes in the microvascular bed of tissue. Hence, fluid intake, distribution and output can be effectively estimated in order to model the fluid map for the whole body 1, whereby facilitating a continuous monitoring of hydration status for the whole body 1 or of a specific location on the body 1.

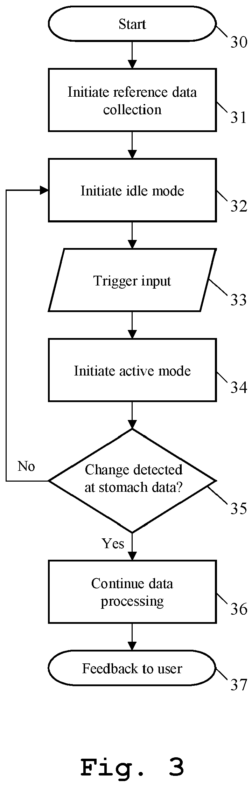

In Fig. 3, an exemplary process flow chart for controlling the sensors is illustrated. The process flow chart corresponds to the implementation where the processing means 13 utilizes a swallowing or fluid intake detection event as a control parameter for the sensors 11₁,11₂,11₃,11₄. After commencing the measurement 30, the processing means 13 initiates 31 the sensors 11₁,11₂,11₃,11₄, preferably in a predefined sequence (e.g., based on a certain health condition to be monitored) and collects data with the sensors 11₁,11₂,11₃,11₄ for approximately 60 seconds in order to gather reference values. Once the reference data are collected, the system 10 maintains an idle mode 32 where only the throat sensor 11₁ is active and is continuously monitoring and the rest of the sensors 11₂,11₃,11₄ are inactive.

If the throat sensor 11₁ detects a swallowing or fluid intake event, it transmits 33 a trigger signal towards the processing means 13. The processing means 13 further generates control signals based on the trigger input in order to sequentially activate the rest of the sensors 11₂,11₃,11₄, thereby initiating an active mode 34. All sensors 11₂,11₃,11₄ are therefore activated in sequence and start to collect data immediately after trigger. However, it is possible that the swallowing event does not correspond to an actual drinking incident, e.g., swallowing without actually drinking water, and therefore the trigger signal would be a false indication for commencing data collection with the sensors 11₂,11₃,11₄.

In order to detect such false positive triggers, the processing means 13 performs additional checks 35 on data collected at stomach. If the collected data do not show any significant discrepancy for a certain period after trigger, for instance, if the stomach sensor does not detect anything within 5-10 minutes after trigger, the measurement is terminated and the system resumes back to its idle mode 32. If the collected data at the stomach show sufficient change within this duration, the trigger is considered as positive trigger and the processing means 13 continues with data processing 36 and with further feedback 37 to the user.

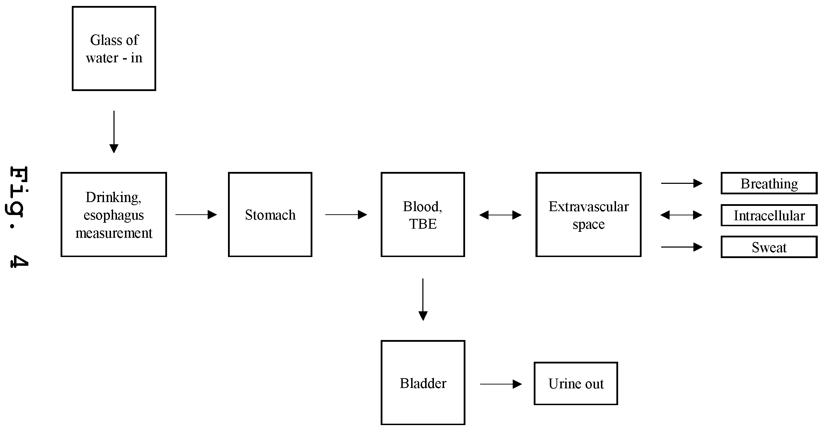

In Fig. 4, an exemplary model for measurement data processing and feedback is illustrated. The model processing is preferably performed in the cloud environment. The rate and clearance constants (e.g., k₁₂ and k_{rel_12}) are in per minute and volumes are in liters. However, the model parameters can be personalized by a user with respect to a standard model. Additionally, the model parameters can be personalized based on calibration or on over time adaptation. The model processing is preferably automated based on a drinking activity, which is used as the calibration event. Bio-impedance measurement at throat (esophagus measurement) confirms the drinking activity where the stomach data is processed to estimate the volume of fluid intake.

With bio-impedance measurement and additional PPG and BP data processing result in the blood volume estimation (total blood equilibrium). Moreover, intra and extracellular volumes can further be estimated from the bio-impedance measurement. In this context, an alternative modeling between blood and extravascular space and between extravascular space and intracellular space can be based on osmotic pressure differences. The fluid output can be estimated from the bladder, e.g., by estimating the volume of fluid loss through urine, as well as from the estimation of fluid loss through sweat.

Standard model processing can be incorporated herein in order to get references and/or thresholds for data collection and processing. For instance, with respect to the delay between fluid intake and distribution, it takes typically 5 minutes for water to show up in the blood after drinking and 11-13 minutes before half of the water is absorbed. Generally, it takes 75-120 minutes before all water is absorbed into the body. The water is detected initially in the central compartment (blood and extravascular with rapid uptake) and next is spread to peripheral compartment (extravascular with slow uptake).

In terms of fluid removal, kidney produces an average of 30-50ml urine per hour, where the normal range per day is 0.6-2.6 liters. This is also vastly dependent on hydration status. Bladder can hold 300-400ml for up to 2-5 hours. Fluid removal through sweat is dependent on any ongoing activity as well as on the ambient temperature. Any field activity result in higher fluid loss through sweat, e.g., playing cricket typically results in a fluid loss of 0.5 liters per hour, playing rugby results in 2.6 liters per hour. Moreover, the average amount of fluid loss for a healthy person during sleep, e.g., through sweat and through exhale, is approximately 25ml per hour.

It is to be noted that, in order to estimate fluid amount (or TBW) fit function using metadata (such as, but not limited to, gender, age and weight) from the subject and data from the bio-impedance measurement as an input to reference fluid measurements. To obtain the bio-impedance parameters, the bio-impedance measurement should be preprocessed and for some of the parameters a human tissue model should be fitted.

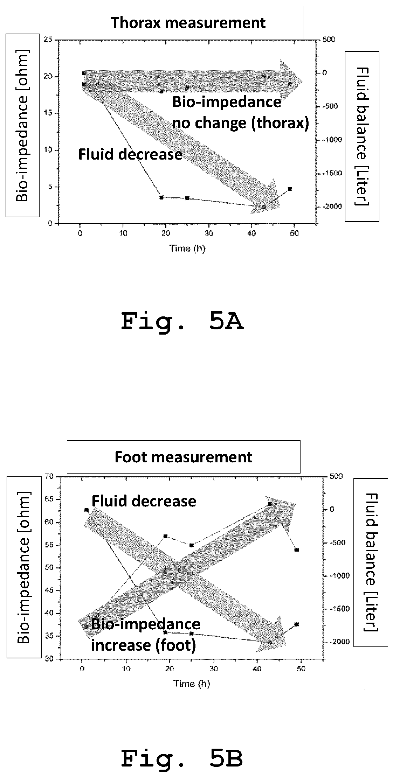

Along Fig. 5A and Fig. 5B, exemplary correlation analysis between fluid balance and bio-impedance for a heart failure patient are illustrated. In particular, Fig. 5A shows the correlation between fluid balance and bio-impedance on thorax, and Fig. 5B shows the correlation between fluid balance and bio-impedance on foot. In both illustrations, the left vertical axis denotes measured bio-impedance in ohm, the right vertical axis denotes fluid balance in liter and the bottom horizontal axis denotes elapsed time in hour.

Heart failure often only affects the left or right side of the heart, but can affect both. In the case of left-sided heart failure, the left ventricle of the heart no longer pumps enough blood around the body. As a result, blood builds up in the pulmonary veins (i.e., fluid accumulated in the lung). In the case of right-sided heart failure, the right ventricle of the heart is too weak to pump enough blood to the lungs. This causes fluid build up in the peripherals. In the case of biventricular heart failure, both sides of the heart are affected, which leads to the combined effects as both left-sided and right-sided heart failure.

Moreover, if the monitoring is performed only on the thorax, e.g., a single/dedicated sensor measurement system, it can have better sensitivity to the fluid in the lung, especially compared to the whole-body bio-impedance, but it cannot detect the fluid balance from the other body part. The inventive solution of having multiple bio-impedance sensors forming a sensor network resolves the foregoing limitation, which can be implemented for monitoring patients' conditions with either one-sided heart failure or biventricular heart failure.

The correlation analysis along Fig. 5A and Fig. 5B are performed on a patient with right-sided heart failure. It can be seen that, for thorax measurement (Fig. 5A), no correlation between fluid balance and measured bio-impedance is present since there is no pulmonary congestion. By contrast, for foot measurement (Fig. 5B), high (negative) correlation between fluid balance and measured bio-impedance is observed, especially due to peripheral congestion. Such high correlation is expected since the patient is suffered from right-sided heart failure. Therefore, only thorax measurement, e.g., with a single or dedicated sensor, is a good solution for left-sided heart failure but not for right-sided heart failure or for biventricular heart failure. A sensor network with multiple sensors can evidently provide more insights about the whole body fluid map.

Furthermore, the presented solution can also be implemented for monitoring patients with chronic kidney disease. Generally, patients with kidney disease accumulate the fluid in their whole body in-between two dialysis sessions. The proposed compartment based measurement approach can provide the information about fluid status of the patient in real-time, so that they know how much they can drink more or how much they have to remove during dialysis. In addition to the current fluid status, fluid intake and output are also monitored continuously for generating complete feedback to the caregiver.

The proposed solution is not limited to patient monitoring with certain health conditions, it can further be implemented for general hydration monitoring for athletes or elderly people. Elderly people or people undergoing extreme sport can experience extreme dehydration and only monitoring the amount of water consumption is not the ultimate solution, since it does not show where the fluid goes after consuming. The proposed solution advantageously model a whole body fluid map using compartmental sensor network in order to monitor drinking, absorbing into the body, increase in total body water, excretion and decrease in total body water. Such an intense model can determine whether the person is in dehydration status or not in a superior manner.

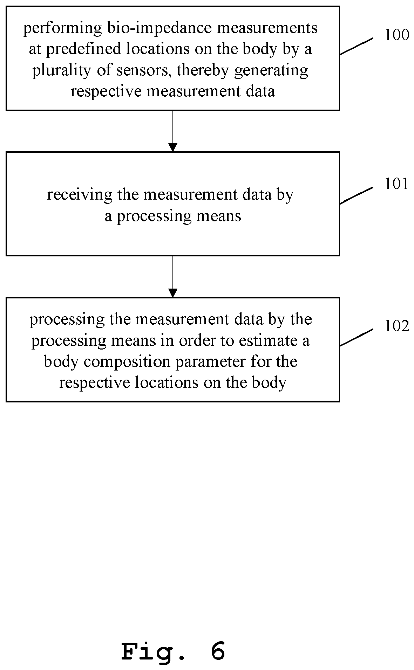

In Fig. 6, an exemplary embodiment of the method according to the second aspect of the invention is illustrated. In a first step 100, bio-impedance measurements are performed by a plurality of sensors at predefined locations on the body, thereby generating respective measurement data. In a second step 101, the measurement data are received by a processing means. In a third step 102, the measurement data are processed by the processing means in order to estimate a body composition parameter for the respective locations on the body.

The embodiments of the present invention can be implemented by hardware, software, or any combination thereof. Various embodiments of the present invention may be implemented by one or more application specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, or the like.

While various embodiments of the present invention have been described above, it should be understood that they have been presented by way of example only, and not limitation. Numerous changes to the disclosed embodiments can be made in accordance with the disclosure herein without departing from the spirit or scope of the invention. Thus, the breadth and scope of the present invention should not be limited by any of the above described embodiments. Rather, the scope of the invention should be defined in accordance with the following claims and their equivalents.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A system (10) for monitoring hydration status of a human body (1) comprising:
a plurality of sensors (11₁, 11₂, 11₃, 11₄) adapted to perform bio-impedance measurements at predefined locations on the body (1), thereby generating respective measurement data; and
a processing means (13) adapted to receive and to process the measurement data in order to estimate a body composition parameter for the respective locations on the body (1),
wherein at least one sensor (11₁) is adapted to perform bio-impedance measurement at the throat region or esophagus region (2) in order to detect a swallowing or fluid intake event.

2. The system according to claim 1,
wherein the processing means (13) is further adapted to control the rest of the sensors (11₂,11₃,11₄) based on the detected swallowing or fluid intake event, and/or
wherein the body composition parameter includes the estimation of fluid volume, fat mass, lean mass or a combination thereof.

3. The system according to claim 1 or 2,
wherein at least one sensor is adapted to perform bio-impedance measurement at a predefined location capable of capturing the impedance changes of the stomach (3), thereby generating measurement data for estimating a fluid volume in the stomach (3).

4. The system according to any of claims 1 to 3,
wherein at least one sensor is adapted to perform bio-impedance measurement at a predefined location capable of capturing the impedance changes of the bladder (7), thereby generating measurement data for estimating a fluid volume in the bladder (7).

5. The system according to any of claims 1 to 4,
wherein the plurality of sensors (11₁, 11₂, 11₃, 11₄) are interchangeable with respect to their respective locations on the body (1), and/or
wherein the plurality of sensors (11₁, 11₂, 11₃, 11₄) are integrated in clothing in order to link the plurality of sensors (11₁, 11₂, 11₃, 11₄) to their respective locations on the body (1).

6. The system according to any of claims 1 to 5,
wherein the system (10) is operable in an idle mode where at least one sensor (11₁) is continuously active and the rest of the sensors (11₂,11₃,11₄) are in sleep mode.

7. The system according to claim 6,
wherein the continuously active sensor (11₁) preferably performs bio-impedance measurement at the throat region or esophagus region (2) and is further adapted to transmit a trigger signal towards the processing means (13) upon detecting a swallowing or fluid intake event.

8. The system according to claim 7,
wherein the processing means (13) is further adapted to generate control signals based on the trigger signal in order to perform wakeup control of the sensors (11₂,11₃,11₄) that are in sleep mode.

9. The system according to any of claims 1 to 8,
wherein the processing means (13) is further adapted to configure the plurality of sensors (11₁, 11₂, 11₃, 11₄) based on predefined time settings in order to define a measurement duration and a measurement rate of recurrence for the respective sensors.

10. The system according to any of claims 1 to 9,
wherein the plurality of sensors (11₁, 11₂, 11₃, 11₄) are further adapted to transmit the respective measurement data to the processing means (13) upon detecting a variation with respect to a reference value or to a value from an immediate preceding measurement.

11. The system according to any of claims 1 to 10,
wherein the system (10) further comprises at least one additional sensor adapted to perform galvanic skin response measurement and/or sweat composition measurement at a predefined location on the body (1), thereby generating measurement data for fluid loss estimation, and/or wherein the system (10) further comprises at least one additional sensor adapted to perform photoplethysmogram measurement at a predefined location on the body (1), thereby generating measurement data in order to increase the accuracy for the fluid estimation.

12. The system according to any of claims 1 to 11,
wherein the processing means (13) is implemented on a cloud environment.

13. The system according to any of claims 1 to 12,
wherein the system (10) further comprises a user interface (15) and whereby the processing means (13) is further adapted to configure the plurality of sensors (11₁, 11₂, 11₃, 11₄) based on control commands received from a user via the user interface (15).

14. A method for monitoring hydration status of a human body (1) comprising the steps of:
performing bio-impedance measurements at predefined locations on the body (1) by a plurality of sensors (11₁, 11₂, 11₃, 11₄), thereby generating respective measurement data; and
receiving and processing the measurement data by a processing means (13) in order to estimate a body composition parameter for the respective locations on the body (1),
wherein at least one sensor (11₁) is adapted to perform bio-impedance measurement at the throat region or esophagus region (2) in order to detect a swallowing or fluid intake event.

15. Computer program with program code means adapted for monitoring hydration status of a human body according to the method of claim 14 when said program runs on a system according to any of claims 1 to 13.
